# DEMANDE DE BREVET EUROPEEN

(11) **EP 1 775 288 A1**
(43) Date de publication de la demande: **18.04.2007**
(21) Numéro de dépôt: 06291573.1
(22) Date de dépôt: 10.10.2006
(51) Int. Cl.: C07D 223/16, A61K 31/55, A61P 9/00

(54) **Forme cristalline § du chlorhydrate de l'ivabradine, son procédé de préparation, et les compositions pharmaceutiques qui la contiennent**

(30) Priorité: 11.10.2005 FR 0510352
(71) Demandeur: Les Laboratoires Servier, 92415 Courbevoie Cedex (FR)
(72) Inventeur: Horvath, Stéphane, 45380 La-Chapelle-Saint-Mesmin (FR); Auguste, Marie-Noëlle, 45000 Orleans (FR); Damien, Gérard, 45130 Meung-sur-Loire (FR)
(74) Mandataire: Giudicelli, Cathy

(57) **Abrégé**

Forme cristalline δ du chlorhydrate de l'ivabradine de formule (I) : 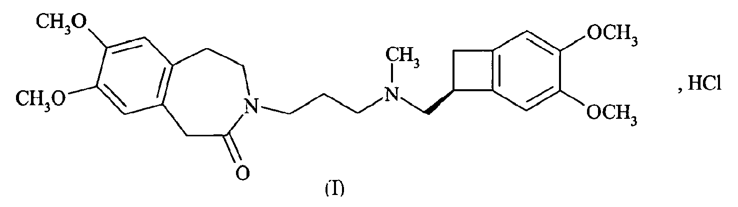 caractérisée par son diagramme de diffraction X sur poudre.

Médicaments.

## Description

La présente invention concerne la forme cristalline δ du chlorhydrate de l'ivabradine de formule (I), son procédé de préparation ainsi que les compositions pharmaceutiques qui la contiennent. 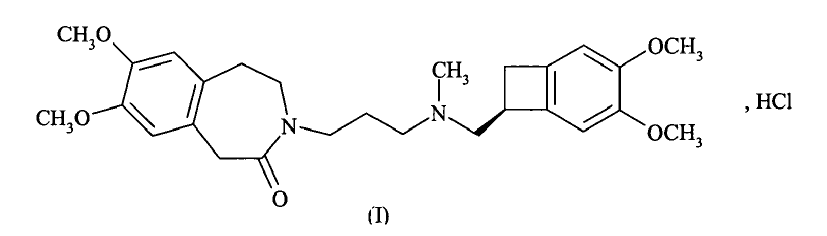 L'ivabradine, ainsi que ses sels d'addition à un acide pharmaceutiquement acceptable, et plus particulièrement son chlorhydrate, ont des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Compte tenu de l'intérêt pharmaceutique de ce composé, il était primordial de l'obtenir avec une excellente pureté. Il était également important de pouvoir le synthétiser selon un procédé facilement transposable à l'échelle industrielle, et notamment sous une forme permettant une filtration et un séchage rapides. Enfin, cette forme devait être parfaitement reproductible, facilement formulée et suffisamment stable pour autoriser son stockage prolongé sans conditions particulières de température, de lumière ou de taux d'oxygène.

Le brevet EP 0534 859 décrit un procédé de synthèse de l'ivabradine et de son chlorhydrate. Cependant, ce document ne précise pas les conditions d'obtention de l'ivabradine sous une forme présentant ces caractéristiques de manière reproductible.

La demanderesse a présentement trouvé qu'un sel particulier de l'ivabradine, le chlorhydrate, pouvait être obtenu sous une forme cristalline bien définie, et présentant des caractéristiques intéressantes de stabilité et de processabilité.

Plus spécifiquement, la présente invention concerne la forme cristalline δ du chlorhydrate de l'ivabradine, caractérisée par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :

| **Raie n°** | **Angle 2 thêta (degrés)** | **Hauteur (coups)** | **Surface (coups x degrés) (Å)** | **FWHM (degrés)** | **Distance inter-réticulaire** |
|---|---|---|---|---|---|
| 1 | 4,1 | 1115 | 110 | 0,1004 | 21,753 |
| 2 | 6,8 | 183 | 145 | 0,8029 | 12,907 |
| 3 | 8,4 | 755 | 75 | 0,1004 | 10,531 |
| 4 | 10,9 | 1104 | 128 | 0,1171 | 8,087 |
| 5 | 12,2 | 195 | 19 | 0,1004 | 7,268 |
| 6 | 14,3 | 569 | 75 | 0,1338 | 6,214 |
| 7 | 14,7 | 1847 | 274 | 0,1506 | 6,013 |
| 8 | 15,3 | 1734 | 315 | 0,184 | 5,802 |
| 9 | 16,3 | 1164 | 154 | 0,1338 | 5,442 |
| 10 | 16,8 | 3420 | 734 | 0,2175 | 5,269 |
| 11 | 17,5 | 790 | 78 | 0,1004 | 5,069 |
| 12 | 17,9 | 3389 | 503 | 0,1506 | 4,960 |
| 13 | 19,2 | 2467 | 407 | 0,1673 | 4,635 |
| 14 | 19,8 | 145 | 29 | 0,2007 | 4,477 |
| 15 | 20,4 | 313 | 52 | 0,1673 | 4,362 |
| 16 | 21,2 | 928 | 169 | 0,184 | 4,198 |
| 17 | 21,7 | 2093 | 414 | 0,2007 | 4,099 |
| 18 | 22,2 | 3850 | 635 | 0,1673 | 4,007 |
| 19 | 22,5 | 576 | 76 | 0,1338 | 3,948 |
| 20 | 23,1 | 1588 | 236 | 0,1506 | 3,855 |
| 21 | 24,8 | 1665 | 247 | 0,1506 | 3,594 |
| 22 | 25,2 | 1212 | 120 | 0,1004 | 3,534 |
| 23 | 25,6 | 1507 | 249 | 0,1673 | 3,477 |
| 24 | 26,7 | 2042 | 303 | 0,1506 | 3,342 |
| 25 | 27,6 | 2281 | 414 | 0,184 | 3,229 |
| 26 | 28,4 | 485 | 96 | 0,2007 | 3,138 |
| 27 | 29,6 | 599 | 99 | 0,1673 | 3,014 |

L'invention s'étend également à un procédé de préparation de la forme cristalline δ du chlorhydrate de l'ivabradine, caractérisé en ce que l'on préchauffe de l'acétonitrile ou un mélange d'acétonitrile et d'eau, on ajoute le chlorhydrate d'ivabradine, on laisse refroidir la solution obtenue à température ambiante et laisse à cette température jusqu'à cristallisation complète, puis on recueille les cristaux formés.
- Dans le procédé de cristallisation selon l'invention, on peut utiliser le chlorhydrate de l'ivabradine obtenu par n'importe quel procédé, par exemple le chlorhydrate de l'ivabradine obtenu par le procédé de préparation décrit dans le brevet EP 0534 859.
- La solution peut être avantageusement ensemencée pendant l'étape de refroidissement.
- L'acétonitrile ou le mélange d'acétonitrile et d'eau est avantageusement préchauffé à une température comprise entre 60°C et la température de reflux, plus préférentiellement à une température comprise entre 65 et 75°C.
- La dilution est préférentiellement supérieure à 15 ml/g, plus préférentiellement comprise entre 50 et 100 ml/g.

L'invention s'étend aussi aux compositions pharmaceutiques renfermant comme principe actif la forme cristalline δ du chlorhydrate de l'ivabradine avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention.

Le spectre de diffraction X sur poudre a été mesuré avec les conditions expérimentales suivantes :
- Diffractomètre PANalytical X'Pert Pro, détecteur X'Celerator, chambre régulée en température,
- Tension 45 KV, intensité 40mA,
- Montage θ-θ,
- Filtre nickel (Kβ),
- Fente de Soller sur le faisceau incident et sur le faisceau diffracté : 0,04 rad,
- Fentes de divergence automatique : longueur irradiée de 10 mm,
- Masque : 10 mm,
- Fente anti-diffusion : 1/2°,
- Mode de mesure : continu de 3° à 30°, avec une incrémentation de 0,017°,
- Temps de mesure par pas : 19,7 s,
- Temps total : 4 min 32 s,
- Vitesse de mesure : 0,108 °/s.
- Température de mesure : ambiante.

### EXEMPLE 1 : Forme cristalline δ du chlorhydrate de l'ivabradine

160 ml d'acétonitrile sont préchauffés à 70°C, puis 2 g du chlorhydrate de l'ivabradine obtenu selon le procédé décrit dans le brevet EP 0534 859 sont ajoutés par portions sous agitation, jusqu'à dissolution complète. La solution est ensuite stockée à température ambiante pendant 2 jours. Les cristaux sont retirés par filtration sous vide et sont étalés sur un plateau de cristallisation.

La teneur en eau du produit obtenu, déterminée par coulométrie, est de 2,8%.

### Diagramme de diffraction X sur poudre :

Le profil de diffraction des rayons X de la poudre (angles de diffraction) de la forme δ du chlorhydrate de l'ivabradine est donné par les raies significatives rassemblées dans le tableau suivant :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,1 | 1115 | 110 | 0,1004 | 21,753 |
| 2 | 6,8 | 183 | 145 | 0,8029 | 12,907 |
| 3 | 8,4 | 755 | 75 | 0,1004 | 10,531 |
| 4 | 10,9 | 1104 | 128 | 0,1171 | 8,087 |
| 5 | 12,2 | 195 | 19 | 0,1004 | 7,268 |
| 6 | 14,3 | 569 | 75 | 0,1338 | 6,214 |
| 7 | 14,7 | 1847 | 274 | 0,1506 | 6,013 |
| 8 | 15,3 | 1734 | 315 | 0,184 | 5,802 |
| 9 | 16,3 | 1164 | 154 | 0,1338 | 5,442 |
| 10 | 16,8 | 3420 | 734 | 0,2175 | 5,269 |
| 11 | 17,5 | 790 | 78 | 0,1004 | 5,069 |
| 12 | 17,9 | 3389 | 503 | 0,1506 | 4,960 |
| 13 | 19,2 | 2467 | 407 | 0,1673 | 4,635 |
| 14 | 19,8 | 145 | 29 | 0,2007 | 4,477 |
| 15 | 20,4 | 313 | 52 | 0,1673 | 4,362 |
| 16 | 21,2 | 928 | 169 | 0,184 | 4,198 |
| 17 | 21,7 | 2093 | 414 | 0,2007 | 4,099 |
| 18 | 22,2 | 3850 | 635 | 0,1673 | 4,007 |
| 19 | 22,5 | 576 | 76 | 0,1338 | 3,948 |
| 20 | 23,1 | 1588 | 236 | 0,1506 | 3,855 |
| 21 | 24,8 | 1665 | 247 | 0,1506 | 3,594 |
| 22 | 25,2 | 1212 | 120 | 0,1004 | 3,534 |
| 23 | 25,6 | 1507 | 249 | 0,1673 | 3,477 |
| 24 | 26,7 | 2042 | 303 | 0,1506 | 3,342 |
| 25 | 27,6 | 2281 | 414 | 0,184 | 3,229 |
| 26 | 28,4 | 485 | 96 | 0,2007 | 3,138 |
| 27 | 29,6 | 599 | 99 | 0,1673 | 3,014 |

### EXEMPLE 2 : Composition pharmaceutique

Formule de préparation pour 1000 comprimés dosés à 5 mg d'ivabradine base:

| | |
|---|---|
| Composé de l'exemple 1 | 5,39 g |
| Amidon de maïs | 20 g |
| Silice colloïdale anhydre | 0,2 g |
| Mannitol | 63,91 g |
| PVP | 10 g |
| Stéarate de magnésium | 0,5 g |

## Revendications

1. Forme cristalline δ du chlorhydrate de l'ivabradine de formule (I) : 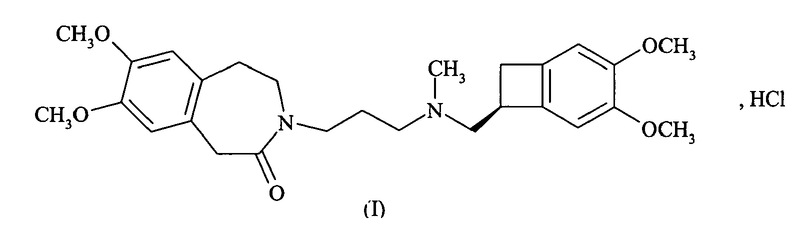 **caractérisée par** le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å):
| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,1 | 1115 | 110 | 0,1004 | 21,753 |
| 2 | 6,8 | 183 | 145 | 0,8029 | 12,907 |
| 3 | 8,4 | 755 | 75 | 0,1004 | 10,531 |
| 4 | 10,9 | 1104 | 128 | 0,1171 | 8,087 |
| 5 | 12,2 | 195 | 19 | 0,1004 | 7,268 |
| 6 | 14,3 | 569 | 75 | 0,1338 | 6,214 |
| 7 | 14,7 | 1847 | 274 | 0,1506 | 6,013 |
| 8 | 15,3 | 1734 | 315 | 0,184 | 5,802 |
| 9 | 16,3 | 1164 | 154 | 0,1338 | 5,442 |
| 10 | 16,8 | 3420 | 734 | 0,2175 | 5,269 |
| 11 | 17,5 | 790 | 78 | 0,1004 | 5,069 |
| 12 | 17,9 | 3389 | 503 | 0,1506 | 4,960 |
| 13 | 19,2 | 2467 | 407 | 0,1673 | 4,635 |
| 14 | 19,8 | 145 | 29 | 0,2007 | 4,477 |
| 15 | 20,4 | 313 | 52 | 0,1673 | 4,362 |
| 16 | 21,2 | 928 | 169 | 0,184 | 4,198 |
| 17 | 21,7 | 2093 | 414 | 0,2007 | 4,099 |
| 18 | 22,2 | 3850 | 635 | 0,1673 | 4,007 |
| 19 | 22,5 | 576 | 76 | 0,1338 | 3,948 |
| 20 | 23,1 | 1588 | 236 | 0,1506 | 3,855 |
| 21 | 24,8 | 1665 | 247 | 0,1506 | 3,594 |
| 22 | 25,2 | 1212 | 120 | 0,1004 | 3,534 |
| 23 | 25,6 | 1507 | 249 | 0,1673 | 3,477 |
| 24 | 26,7 | 2042 | 303 | 0,1506 | 3,342 |
| 25 | 27,6 | 2281 | 414 | 0,184 | 3,229 |
| 26 | 28,4 | 485 | 96 | 0,2007 | 3,138 |
| 27 | 29,6 | 599 | 99 | 0,1673 | 3,014 |

2. Procédé de préparation de la forme cristalline δ du chlorhydrate de l'ivabradine selon la revendication 1, **caractérisé en ce que** l'on préchauffe de l'acétonitrile ou un mélange d'acétonitrile et d'eau, on ajoute le chlorhydrate d'ivabradine, on laisse refroidir la solution obtenue à température ambiante et laisse à cette température jusqu'à cristallisation complète, puis on recueille les cristaux formés.

3. Procédé selon la revendication 2, **caractérisé en ce que** la solution du chlorhydrate de l'ivabradine est ensemencée pendant l'étape de refroidissement.

4. Composition pharmaceutique contenant comme principe actif la forme cristalline δ du chlorhydrate de l'ivabradine selon la revendication 1, en combinaison avec un ou plusieurs véhicules inertes, non toxiques et pharmaceutiquement acceptables.

5. Utilisation de la forme cristalline δ du chlorhydrate de l'ivabradine selon la revendication 1, pour la fabrication de médicaments utiles comme bradycardisants.

6. Utilisation de la forme cristalline δ du chlorhydrate de l'ivabradine selon la revendication 1, pour la fabrication de médicaments utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.
